# EUROPEAN PATENT SPECIFICATION

(11) **EP 3 781 543 B1**
(45) Date of publication and mention of the grant of the patent: **27.09.2023**
(21) Application number: 19716915.4
(22) Date of filing: 15.04.2019
(51) Int. Cl.: C07C 46/08, C07C 50/04, C07C 50/12, C07C 50/02

(54) **OXIDATION OF ALKYLATED P-HYDROQUINONES IN AQUEOUS SOLUTIONS BY OXYGEN**
OXIDATION VON ALKYLIERTEN P-HYDROCHINONEN IN WÄSSRIGEN LÖSUNGEN DURCH SAUERSTOFF
OXYDATION DE P-HYDROQUINONES ALKYLÉS DANS DES SOLUTIONS AQUEUSES PAR OXYGÈNE

(30) Priority: 17.04.2018 EP 18167715
(43) Date of publication of application: 24.02.2021
(73) Proprietor: DSM IP Assets B.V., 6411 TE Heerlen (NL)
(72) Inventor: BONRATH, Werner, 4303 Kaiseraugst (CH); MUELLER, Marc-André, 4303 Kaiseraugst (CH); NETSCHER, Thomas, 4303 Kaiseraugst (CH)
(74) Representative: Dux, Roland
(86) International application number: PCT/EP2019/059588
(87) International publication number: WO 2019/201819

(56) References cited:
- US-A- 3 663 578
- US-A- 5 712 416

## Description

### Technical Field

The present invention relates to the oxidation of alkylated p-hydroquinones.

### Background of the invention

Alkylated p-hydroquinones and alkylated p-benzoquinones are important intermediates in a series of industrial applications. Particularly, they are used in the synthesis of tocopherols.

The oxidation of alkylated p-hydroquinones to obtain the respective alkylated p-benzoquinones is particularly interesting for the industry.

Oxidation of phenols per se is known to the person skilled in the art. For example, EP 0 659 727 A1 discloses a process for the preparation of benzoquinones by oxidation of phenols in the presence of a complex of iron, manganese or chromium with tetraaza[14]annulene as polydentate complexing ligand.

M. Shimizu, in Tetrahedron Lett., 1989, 30, 471-474, discusses the oxidation of a phenol carried out by H₂O₂ in the presence of a heteropolyacid.

WO 2015/169898 A1 discloses the oxidation of 2,6-dimethylphenol to 2,6-dimethylbenzoquinone by means of O₂ in the presence of a copper salt.

WO 2015/000767 A1 discloses the oxidation of 2,3,6-trimethylphenol to 2,3,6-trimethylbenzoquinone by means of O₂ in the presence of a copper salt and a secondary aliphatic acyclic alcohol with 6 or more carbon atoms.

These documents, however, all relate to the oxidation of phenols, i.e. compounds carrying only one hydroxyl group at the aromatic ring, and not to the oxidation of hydroquinones, i.e. carrying two hydroxyl groups in para positions at the aromatic ring.

R.G. Jones et al. in J. Am. Chem. Soc. 1945, 67, 1034-1035 disclose the preparation of 2,5-dihydroxyquinone by oxidation of hydroquinone in highly concentrated (50 %) sodium hydroxide solution with 30 % hydrogen peroxide. These harsh reaction conditions, however, lead to significant safety risks.

US 3,663,578 discloses the oxidation of alkyl-substituted hydroxyquinones to the corresponding p-benzoquinones in a two phase system and the presence of copper chloride as catalyst. Hence, this document discloses large quantities of organic solvent, such as cyclohexane. Due to presence of large amounts of organic solvents, used as one of the two phases, the disclosed process is very critical in view of safety which is even pronounced in the presence of oxygen in said process. Furthermore, US 3,663,578 discloses that this process is performed at a pH from 0.5 to 4, particularly from 1.0 to 3.0. Such low pH is very critical because of risk corrosion of the equipment used or health risk for the employees involved in said production process. Finally, major quantities of base need to be employed when neutralizing the residues after termination of the reaction. This is economically and environmentally very disadvantageous. The combined risk of corrosion and hazards of fire or explosion is, however, unacceptably high for a production process, particularly in an industrial scale.

EP 0 659 727 A1 discloses the oxidation of phenols by hydrogen peroxide or oxygen in the presence of an oxygen-transferring catalyst selected from the group consisting of iron, manganese and chromium tetraaza[14]annulenes to the respective benzoquinones in a of large amount of diluents, particularly acetic acid.

US 3,663,578 discloses a catalytic oxidation of alkyl-substituted hydroquinones to the corresponding p-benzoquinones by air in a liquid mixed phase, i.e. the presence of water and an organic solvent using a Cu(II) salt as catalyst.

Hydrogen peroxide is an oxidizing agent which needs special care in handling.

Organic diluents, and particularly acetic acid, are inflammable organic liquids and are as a result thereof critical in fire hazards or explosions. The use of large amounts of acetic acid in presence of oxygen is not only disadvantageous in view of fire hazard but also in view of corrosion hazard.

Furthermore, the solubility of oxygen gas in organic solvents is rather limited. A good solubility of molecular oxygen, however, is very essential for an efficient and cost-friendly oxidation.

Metal complexes having organic ligands, particularly polydentate ligands, are known. However, such complexes are very expensive and commercially not readily available. This is particularly true for complexes of tetraaza[14]annulenes. Furthermore, is their eco-impact also to be assessed rather as critical. Finally, it has been shown, that metal complexes are only of limited interest for oxidation reactions as the metal complexes, particularly the ligands involved, are prone to be oxidized themselves. This of course is very disadvantageous in view of efficiency and re-cyclability of these metal compounds.

Finally, the solubility of the metal complexes in water is a very important limiting factor for the oxidation to occur in a cost-efficient way.

All these factors show, that there is a great need in industry for to offer a save, economically friendly and cost-efficient method of oxidation of alkylated p-hydroquinones to the corresponding alkylated p-benzoquinones.

### Summary of the invention

The present invention offers an improved process of oxidation of alkylated p-hydroquinones to the corresponding alkylated p-benzoquinones.

It has been shown that this process leads to a high yield and excellent selectivity and reaction rate. The invention enables a very safe production particularly in regard to fire hazards. By using oxygen, particularly in form of air, as oxidizing agent the invention can offer an ecologically friendly and particular inexpensive and safe oxidation process.

Particularly, it has been shown that the manufacturing process is also suitable for aqueous systems at very low concentrations of the alkylated p-hydroquinones.

Further aspects of the invention are subject of further independent claims. Particularly preferred embodiments are subject of dependent claims.

### Detailed description of the invention

In a first aspect, the present invention relates to a process of manufacturing a compound of formula (I) 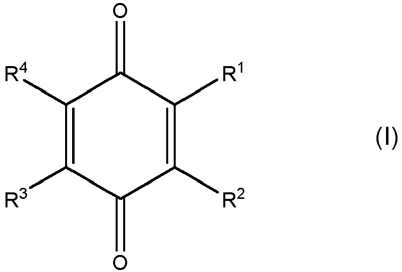 by oxidation of a compound of formula (II) 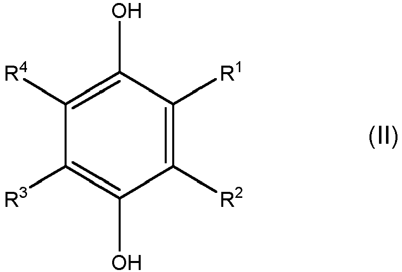
using oxygen or a mixture comprising oxygen and an inert gas in the presence of a transition metal salt, which does not comprise any polydentate ligand bound to the metal ion, and an organic acid and water; wherein R¹, R², R³ and R⁴ independently represent either H or a C₁₋₄-alkyl group or R¹ and R² form together a divalent carbon chain to yield a 6-membered saturated or aromatic ring;
with the proviso that at least one of the residues R¹, R², R³ and R⁴ is different from H,
characterized in that an inert organic solvent is not present or present in an amount not exceeding 3 % by weight, preferably not exceeding 1 % by weight, during the oxidation;
and that that under the conditions of the reaction, the inert gas or the inert solvent does not undergo any chemical reaction or lead to any change of pH.

For sake of clarity, some terms as been used in the present document are defined as follows:
In the present document, a "C_{x-y}-alkyl" group is an alkyl group comprising x to y carbon atoms, i.e., for example, a C₁₋₃-alkyl group is an alkyl group comprising 1 to 3 carbon atoms. The alkyl group can be linear or branched. For example -CH(CH₃)-CH₂-CH₃ is considered as a C₄-alkyl group.

In case identical labels for symbols or groups are present in several formulae, in the present document, the definition of said group or symbol made in the context of one specific formula applies also to other formulae which comprises the same said label.

In the present document, a "transition metal salt" is a salt of a metal ion of the groups 3-12 of the periodic table and does not comprise any polydentate ligand bound to the metal ion.

The term "inert", as used in this document in describing a solvent or gas, means that under the conditions of the reaction said solvent or gas does not undergo any chemical reaction or lead to any change of pH.

The term "essentially no inert organic solvent" as used in the present documents means that ideally no inert organic solvents are present at all. However, "essentially no inert organic solvent" means also that only minor traces in an amount not exceeding 3 % by weight, preferably not exceeding 1 % by weight, can be present. Large amounts of inert solvents, however, would negatively impact the present invention.

The groups R¹, R², R³ and R⁴ independently represent either H or a C₁₋₄-alkyl group or R¹ and R² form together a divalent carbon chain to yield a 6-membered saturated or aromatic ring with the proviso that at least one of the residues R¹, R², R³ and R⁴ is different from H.

In one embodiment, R¹, R², R³ and R⁴ independently represent either H or a C₁₋₄-alkyl group with the proviso that at least one of the residues R¹, R², R³ and R⁴ is different from H.

Particularly, R¹, R², R³ and R⁴ independently represent either H or CH₃. 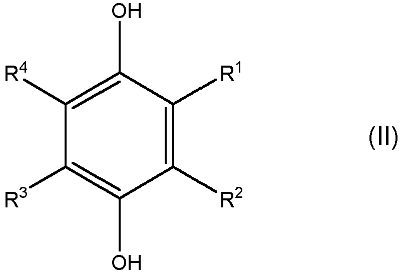 Preferred are the following combinations of R¹, R², R³ and R⁴:
- R⁴ = H and R¹ = R² = R³ = C₁₋₄-alkyl group; particularly CH₃
- R⁴ = R² = H and R¹ = R³ = C₁₋₄-alkyl group; particularly CH₃
- R⁴ = R³ = H and R¹ = R² = C₁₋₄-alkyl group; particularly CH₃
- R⁴ = R³ = R² = H and R¹ = C₁₋₄-alkyl group; particularly CH₃
- R³ = R² = H and R¹ = R⁴ = C₁₋₄-alkyl group; particularly CH₃.

Preferred is that R⁴=H and R¹ = R² = R³ = C₁₋₄-alkyl group; particularly R¹ = R² = R³ = CH₃.
Most preferred is, therefore, the combination of R⁴ = H and R¹ = R² = R³ = CH₃.

In other words, the most preferred compound of formula (I) is 2,3,5-trimethylbenzoquinone (=2,3,5-trimethylcyclohexa-2,5-diene-1,4-dione) and the most preferred compound of formula (II) is 2,3,5-trimethylhydroquinone (=2,3,5-trimethylbenzene-1,4-diol).

The compound of formula (II) is known to the person skilled in the art and easily accessible.

In another embodiment, R¹ and R² form together a divalent carbon chain to yield a 6-membered saturated or aromatic ring and R³ and R⁴ independently represent either H or a C₁₋₄-alkyl group.

Preferably, R¹ and R² form together a divalent carbon chain to yield a 6-membered aromatic ring.

In this embodiment, preferably the compound of formula (II) is a compound of formula (II-A) or (II-B)

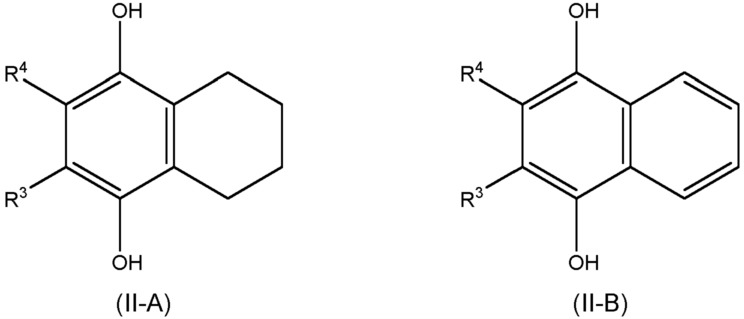

Accordingly, the preferred compound of formula (I) for this embodiment is a compound of formula (I-A) or (I-B).

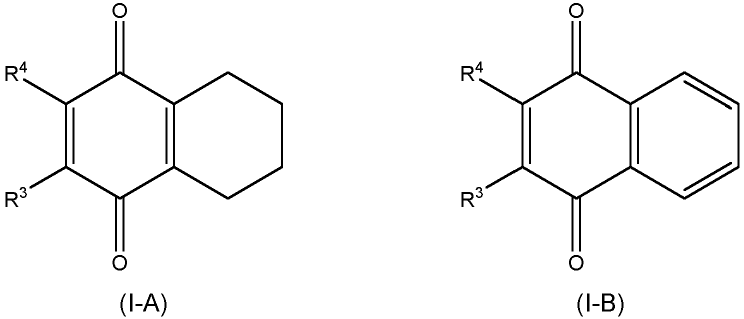

It is preferred that for this embodiment, compound of formula (I) is a compound of (I-B) and compound of formula (II) is a compound of formula (II-B).

Preferably, compound of formula (I-B) is 2-methyl-1,4-naphthoquinone (also called menadione [58-27-5], vitamin K3) which is an important intermediate for the synthesis of vitamins K1 and K2.

The compound of formula (II) is present in an amount of typically 0.3 to 10 % by weight, particularly 0.8 to 5.0 % by weight, preferably 0.8 - 2.5 % by weight, based on the weight of the complete reaction mixture at the beginning of the oxidation.

The above process is carried out in the presence of a transition metal salt which does not comprise any polydentate ligand bound to the metal ion. Said transition metal salt is preferably selected from the group consisting of Ce, Mn, Fe, Cu and Zn, particularly is selected from the group consisting of Mn, Fe, Cu and Zn.

Preferably, the transition metal salt is a salt of Mn(II), Mn(IV), Fe(II), Fe(III), Cu(I), Cu(II) or Zn(II), particularly of Fe(II) or Fe(III). Most preferred is that the transition metal salt is a salt of Fe(II) or Fe(III). Ferric salts (Fe(III)) are preferred over ferrous salts (Fe(II)). It has been shown that ferrous salts give slightly lower yields than ferric salts under the same conditions.

The transition metal salt is preferably soluble in water. Particularly, the solubility of the transition metal salt in water is at least so high that it forms a clear solution at the temperature and amounts of water used for the oxidation reaction.

The transition metal salt is preferably a sulphate, chloride, carboxylate, particularly an acetate, of the transition metal, particularly of a sulphate, chloride, carboxylate, particularly an acetate, of Mn(II), Mn(IV), Fe(II), Fe(III), Cu(I), Cu(II) or Zn(II).

The amount of transition metal salt is present at the beginning of the oxidation in an amount of at least 0.02 mol-%, particularly in an amount of 0.02 - 2 mol-%, more particularly in an amount of 0.3 - 1.0 mol-%, based on the amount of the compound of formula (II).

The transition metal salt can be recycled and be reused.

The process is usually carried out at a temperature of from 40°C to 95°C, particularly between 50°C and 85°C, preferably between 55°C and 70°C.

The above process is carried out in the presence of an organic acid. Preferably, the organic acid is selected from the group consisting of acetic acid, adipic acid, lactic acid, oxalic acid, citric acid, particularly acetic acid.

The amount of organic acid added is in an amount that the pH of the reaction mixture is in the range of 4.8 -6.8, particularly 5 - 6.8, preferably 5.5 - 6.2.

It is preferred that the amounts of organic acid added is 0.15 - 3.5 mol-equivalent, preferably 1.5 -2.5 mol-equivalent, based on the amount of formula (II).

It is important to stress that in the present invention only small amounts of any organic acids are used. The reaction mixtures of the present inventions are advantageous in view of handling, particularly in view of flammability, which is present in the state of the art technology when reaction mixtures are handled in the presence of large amounts of highly volatile organic substances, such as solvents or organic acids, in the presence of oxygen or oxygen-containing gas mixtures.

Furthermore, performing the reaction at a very low pH, particularly at a pH of lower than 3.5, or even lower than 3, is very disadvantageous due to the risk of corrosion of the equipment used or health risk for the employees involved in said production process. Finally, when the reaction would be made under strongly acid condition, major quantities of base would be needed when neutralizing the residues after termination of the reaction, which would be, therefore also very disadvantageous from an economic and/or environmental point of view. With the present invention, it has been observed that the reaction excellently proceeds also at conditions at slightly acid pH or even at neutral pH.

Therefore, it is preferred that the oxidation takes place at a pH of 5 - 6.8, particularly of 5.5 - 6.2.

The oxidation of the compound of formula (II) is carried out by means of oxygen or a mixture comprising oxygen and an inert gas.

Oxygen is a very suitable oxidizing agent as it does not introduce any contaminants originating from the oxidizing agent into the product which might be critical in view of safety and ecology.

Therefore, in one embodiment, molecular oxygen (O₂) can be used as oxidizing agent.

In another embodiment, a mixture comprising oxygen and an inert gas is used as oxidizing agent. It is preferred that the amount of oxygen in such a mixture comprising oxygen and an inert gas is at least 15 % by volume, particularly at least 20 % by volume. Such a mixture may, for example, be a binary mixture such as a mixture oxygen/nitrogen or oxygen/argon or alike. Said mixture can consist of or comprise two or more inert gases. It is particularly preferred to use air as such a mixture comprising oxygen and an inert gas.

It is preferred that that the oxidizing agent is O₂ in the form of air or pure oxygen. Most preferred the oxidizing agent is O₂ in the form of pure oxygen.

Oxygen and particularly air are very cheap oxidizing agents.

The above process is carried out in the presence of water. It has been shown that the oxidation can be carried out under conditions where the amount of water is considerable.

Particularly, it has been found that oxidation of the compound of formula (II) is efficient at even very high amounts of water.

It has been found that the present invention is particularly suitable for oxidation of compound of formula (II) where the amount of the amount of water is more than 90 % by weight, preferably more than 95 % of the reaction mixture.

Hence, it has been found that the present invention offers a very valuable method for the oxidation of compound of formula (II) also in very diluted forms of compound of formula (II) in aqueous systems as they occur for example in work-up of aqueous washing or extractions during productions based on compound of formula (I) or (II) or derivatives thereof.

The above process is carried out so that essentially no inert organic solvent is present during the oxidation. The presence of inert organic solvents, particularly in significant amounts would be negatively impacting the present invention. Inert organic solvents are negatively influencing as they would lead to problems in the solubility of the transition metal salts. Furthermore, their high volatility and flammability would significantly increase the fire hazard.

Particularly, the oxidation is carried out in such a manner that the sum of the weight of
a) the compound of formula (II)
b) oxygen
c) the transition metal salt, which does not comprise any polydentate ligand bound to the metal ion
d) the organic acid
e) water
is more than 95 %, preferably more than 98%, more preferably more than 99 %, of the weight of the complete reaction mixture at the beginning of the oxidation.

In a preferred way of oxidation, a mixture of at least the compound of formula (II), water and the transition metal salt and the organic acid is prepared and oxygen or a mixture comprising oxygen and an inert gas, respectively, is introduced directly or indirectly into said mixture. The flow rate of oxygen or a mixture comprising oxygen and an inert gas, respectively, can vary and depends on the size of the reaction vessel.

It is preferred that the oxidation is carried out in the absence of any complexing agent or any complex having bound a complexing agent as ligand.

The oxidation can take place at ambient pressure or under pressure. It is preferred that the oxidation takes place under pressure, particularly under a pressure of more than 2 bars, preferably more than 3 bars.

The above process is preferably carried out in a continuous way, i.e. that the process is preferably a continuous process. In this embodiment, the process is carried out in a suitable reaction vessel, known per se for continuous reactions.

It has been shown that the process described above leads to a high yield and excellent selectivity and reaction rate. It enables offering a very safe production process. Particularly the fire hazards are strongly reduced in comparison with processes known from the state of the art, particularly with processes using large amounts of organic diluents. The process uses oxygen, particularly in form of air, as oxidizing agent. This leads to the advantage that the present oxidation process is ecologically friendly and particular inexpensive and safe.

Particularly, it has been shown that the process is also suitable for aqueous systems in very low concentrations of the alkylated p-hydroquinones.

### Examples

The present invention is further illustrated by the following experiments.

### General procedure for the oxidation of 2,3,5-trimethylhydroauinone (TMHQ)

An autoclave was charged with H₂O (99.0 mL) and nitrogen was bubbled through for 10 minutes. The equipment was evacuated and back-filled with nitrogen three-times and 2,3,5-trimethylhydroquinone (TMHQ) (1.00 g, 6.45 mmol, 1.00 eq) was added. An aqueous NaOH solution (10%, 5.00 mL) was added to the suspension to adjust the pH value to ~12 and the reaction mixture was stirred for 15 min until all TMHQ had dissolved. The corresponding amount of iron-salt (Fe₂(SO₄)₃×6H₂O) as indicated in table 1 was added and the reaction mixture was warmed to 60°C. The respective amount of acetic acid (as indicated in table 1) and HCl (25%, 1.4 mL) was added to adjust the pH to ~6. Then the autoclave was pressurized with oxygen or air. The reaction was run under stirring and under the pressure and time as indicated in table 1 and allowed to cool to room temperature. After release of the pressure, the reaction mixture was extracted with toluene (2×50 mL). The combined organic layers were washed with H₂O (1×30 mL) and concentrated at 50°C/25 mbar to obtain a yellow liquid being identified to be 2,3,5-trimethylbenzoquinone (TMQ). The obtained yields for TMQ have been compiled in table 1.

**Table 1. Oxidation of TMHQ to TMQ by air or oxygen.**

| **Example^{a)}** | **Oxidant** | **Pressure [bar]** | **Fe [mol-%]** | **Time [h]** | **Yield TMQ [%]** |
|---|---|---|---|---|---|
| ***Ref.1*** | Air | 1 | -- | 2¼ | 11.7 |
| ***1*** | Air | 1 | 0.3 | 2¼ | 78.2 |
| ***2*** | Air | 1 | 0.15 | 2¼ | 67.0 |
| ***3*** | Air | 1 | 0.08 | 2¼ | 63.2 |
| ***4*** | Air | 1 | 0.3 | 1 | 56.7 |
| ***5*** | Air | 1 | 0.6 | 1 | 58.6 |
| ***6*** | Air | 1 | 0.3 | 4½ | 84 |
| ***7*** | Air | 1 | 0.3 | 2 | 23 |
| ***8*** | Air | 4 | 0.3 | 2 | 59 |
| | | | | | |
| ***9*** | O₂ | 4 | 0.3 | 2 | 85 |
| ***10***^{b)} | O₂ | 4 | 0.3 | 2 | 90 |
| ***11***^{c)} | O₂ | 4 | 0.3 | 2 | 74 |

| | | | | | |
|---|---|---|---|---|---|
| ^{a)} 0.2 eq AcOH were used except in example 10 and 11. ^{b)} 2.2 eq AcOH were used. ^{c)} 2.9 eq AcOH were used. | | | | | |

## Claims

1. A process of manufacturing a compound of formula (I) 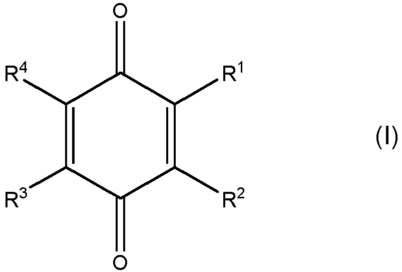 by oxidation of a compound of formula (II) 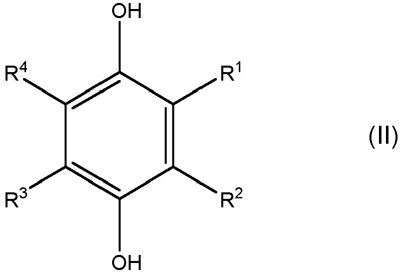
using oxygen or a mixture comprising oxygen and an inert gas in the presence of a transition metal salt, which does not comprise any polydentate ligand bound to the metal ion, and an organic acid and water; wherein R¹, R², R³ and R⁴ independently represent either H or a C₁₋₄-alkyl group or R¹ and R² form together a divalent carbon chain to yield a 6-membered saturated or aromatic ring;
with the proviso that at least one of the residues R¹, R², R³ and R⁴ is different from H,
**characterized in that** an inert organic solvent is not present or present in an amount not exceeding 3 % by weight, preferably not exceeding 1 % by weight, during the oxidation;
and that that under the conditions of the reaction, the inert gas or the inert solvent does not undergo any chemical reaction or lead to any change of pH.

2. The process according to claim 1, **characterized in that** R⁴=H and
R¹ = R² = R³ = C₁₋₄-alkyl group; particularly R¹ = R² = R³ = CH₃.

3. The process according to any one of the preceding claims, **characterized in that** the transition metal is selected from the group consisting of Ce, Mn, Fe, Cu and Zn, particularly is selected from the group consisting of Mn, Fe, Cu and Zn.

4. The process according to any one of the preceding claims, **characterized in that** the transition metal salt is a salt of Mn(II), Mn(IV), Fe(II), Fe(III), Cu(I), Cu(II) or Zn(II), particularly of Fe(II) or Fe(III).

5. The process according to any one of the preceding claims, **characterized in that** the organic acid is selected from the group consisting of acetic acid, adipic acid, lactic acid, oxalic acid, citric acid, particularly acetic acid.

6. The process according to any one of the preceding claims, **characterized in that** the amount of water is more than 90 % by weight, preferably more than 95 % of the reaction mixture.

7. The process according to any one of the preceding claims, **characterized in that** the sum of the weight of
a) the compound of formula (II)
b) oxygen
c) the transition metal salt, which does not comprise any polydentate ligand bound to the metal ion
d) the organic acid
e) water
is more than 95 %, preferably more than 98%, more preferably more than 99 %, of the weight of the complete reaction mixture at the beginning of the oxidation.

8. The process according to any one of the preceding claims, **characterized in that** the compound of formula (II) is present in an amount of 0.3 to 10 % by weight, particularly 0.8 to 5.0 % by weight, preferably 0.8 - 2.5 % by weight, based on the weight of the complete reaction mixture at the beginning of the oxidation.

9. The process according to any one of the preceding claims, **characterized in that** the transition metal of the transition metal salt is present at the beginning of the oxidation in an amount of at least 0.02 mol-%, particularly in an amount of 0.02 - 2 mol-%, more particularly in an amount of 0.3 - 1.0 mol-%, based on the amount of the compound of formula (II).

10. The process according to any one of the preceding claims, **characterized in that** the oxidation takes place at a pH of 5 - 6.8, particularly of 5.5 - 6.2.

11. The process according to any one of the preceding claims, **characterized in that** the oxidizing agent is O₂ in the form of air or pure oxygen, preferably in the form of pure oxygen.

12. The process according to claim 10, **characterized in that** the oxidation takes place under pressure, particularly under a pressure of more than 2 bars, preferably more than 3 bars.

13. The process according to any one of the preceding claims, **characterized in that** the process is a continuous process.

## Patentansprüche

1. Verfahren zur Herstellung einer Verbindung der Formel (I) 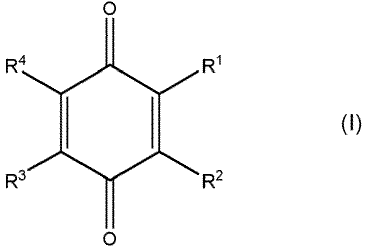 durch Oxidation einer Verbindung der Formel (II) 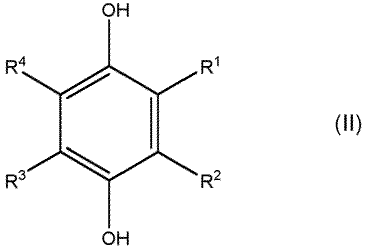
unter Verwendung von Sauerstoff oder einer Mischung, die Sauerstoff und ein Inertgas umfasst,
in Gegenwart von einem Übergangsmetallsalz, das keinen an das Metallion gebundenen mehrzähnigen Liganden umfasst, und einer organischen Säure und Wasser;
wobei R¹, R², R³ und R⁴ unabhängig voneinander für H oder eine C₁₋₄-Alkylgruppe stehen oder R¹ und R² zusammen eine zweiwertige Kohlenwasserstoffkette unter Erhalt eines 6-gliedrigen gesättigten oder aromatischen Rings bilden;
mit der Maßgabe, dass mindestens einer der Reste R¹, R², R³ und R⁴ von H verschieden ist,
**dadurch gekennzeichnet, dass** während der Oxidation kein inertes organisches Lösungsmittel vorliegt oder ein inertes organisches Lösungsmittel in einer Menge von nicht mehr als 3 Gew.-%, vorzugsweise nicht mehr als 1 Gew.-%, vorliegt;
und dass das Inertgas oder das inerte Lösungsmittel unter den Bedingungen der Reaktion keine chemische Reaktion eingeht oder zu einer Änderung des pH-Werts führt.

2. Verfahren nach Anspruch 1, **dadurch gekennzeichnet, dass** R⁴ = H und R¹ = R² = R³ = C₁₋₄-Alkylgruppe; insbesondere R¹ = R² = R³ = CH₃.

3. Verfahren nach einem der vorhergehenden Ansprüche, **dadurch gekennzeichnet, dass** das Übergangsmetall aus der Gruppe bestehend aus Ce, Mn, Fe, Cu und Zn ausgewählt ist und insbesondere aus der Gruppe bestehend aus Mn, Fe, Cu und Zn ausgewählt ist.

4. Verfahren nach einem der vorhergehenden Ansprüche, **dadurch gekennzeichnet, dass** es sich bei dem Übergangsmetallsalz um ein Salz von Mn(II), Mn(IV), Fe(II), Fe(III), Cu(I), Cu(II) oder Zn(II), insbesondere von Fe(II) oder Fe(III), handelt.

5. Verfahren nach einem der vorhergehenden Ansprüche, **dadurch gekennzeichnet, dass** die organische Säure aus der Gruppe bestehend aus Essigsäure, Adipinsäure, Milchsäure, Oxalsäure, Citronensäure, insbesondere Essigsäure, ausgewählt wird.

6. Verfahren nach einem der vorhergehenden Ansprüche, **dadurch gekennzeichnet, dass** die Wassermenge mehr als 90 Gew.-%, vorzugsweise mehr als 95 Gew.-%, der Reaktionsmischung ausmacht.

7. Verfahren nach einem der vorhergehenden Ansprüche, **dadurch gekennzeichnet, dass** die Summe des Gewichts von
a) der Verbindung der Formel (II),
b) Sauerstoff,
c) dem Übergangsmetallsalz, das keinen an das Metallion gebundenen mehrzähnigen Liganden umfasst,
d) der organischen Säure,
e) Wasser
mehr als 95 %, vorzugsweise mehr als 98 %, weiter bevorzugt mehr als 99 %, des Gewichts der kompletten Reaktionsmischung zu Beginn der Oxidation beträgt.

8. Verfahren nach einem der vorhergehenden Ansprüche, **dadurch gekennzeichnet, dass** die Verbindung der Formel (II) in einer Menge von 0,3 bis 10 Gew.-%, insbesondere 0,8 bis 5 Gew.-%, vorzugsweise 0,8-2,5 Gew.-%, bezogen auf das Gewicht der kompletten Reaktionsmischung zu Beginn der Oxidation, vorliegt.

9. Verfahren nach einem der vorhergehenden Ansprüche, **dadurch gekennzeichnet, dass** das Übergangsmetall des Übergangsmetallsalzes zu Beginn der Oxidation in einer Menge von mindestens 0,02 Mol-%, insbesondere in einer Menge von 0,02-2 Mol-%, spezieller in einer Menge von 0,3-1,0 Mol-%, bezogen auf die Menge der Verbindung der Formel (II), vorliegt.

10. Verfahren nach einem der vorhergehenden Ansprüche, **dadurch gekennzeichnet, dass** die Oxidation bei einem pH-Wert von 5-6,8, insbesondere von 5,5-6,2, stattfindet.

11. Verfahren nach einem der vorhergehenden Ansprüche, **dadurch gekennzeichnet, dass** es sich bei dem Oxidationsmittel um O₂ in Form von Luft oder reinem Sauerstoff, vorzugsweise in Form von reinem Sauerstoff, handelt.

12. Verfahren nach Anspruch 10, **dadurch gekennzeichnet, dass** die Oxidation unter Druck stattfindet, insbesondere unter einem Druck von mehr als 2 bar, vorzugsweise mehr als 3 bar.

13. Verfahren nach einem der vorhergehenden Ansprüche, **dadurch gekennzeichnet, dass** es sich bei dem Verfahren um ein kontinuierliches Verfahren handelt.

## Revendications

1. Procédé de fabrication d'un composé de formule (I) 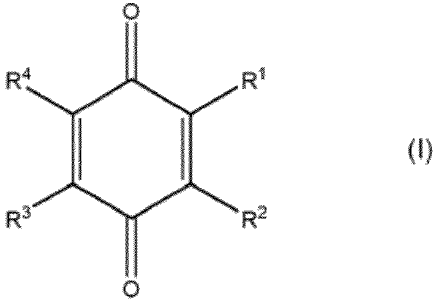 par oxydation d'un composé de formule (II) 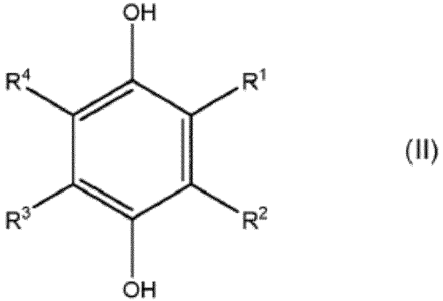
en utilisant de l'oxygène ou un mélange comprenant de l'oxygène et un gaz inerte en la présence d'un sel de métal de transition, qui ne comprend aucun ligand polydentate lié à l'ion métallique, et un acide organique et de l'eau ;
R¹, R², R³ et R⁴ représentant indépendamment soit H, soit un groupe alkyle en C₁₋₄, soit R¹ et R² formant ensemble une chaîne carbonée divalente pour donner un cycle saturé ou aromatique à 6 chaînons ;
à la condition qu'au moins l'un des radicaux R¹, R², R³ et R⁴ soit différent de H,
**caractérisé en ce qu'**un solvant organique inerte n'est pas présent ou est présent en une quantité ne dépassant pas 3 % en poids, préférablement ne dépassant pas 1 % en poids, pendant l'oxydation ; et **en ce que** dans les conditions de la réaction, le gaz inerte ou le solvant inerte ne subit aucune réaction chimique ou ne mène à aucun changement de pH.

2. Procédé selon la revendication 1, **caractérisé en ce que** R⁴ = H et R¹ = R² = R³ = groupe alkyle en C₁₋₄ ; particulièrement R¹ = R² = R³ = CH₃.

3. Procédé selon l'une quelconque des revendications précédentes, **caractérisé en ce que** le métal de transition est choisi dans le groupe constitué par Ce, Mn, Fe, Cu et Zn, particulièrement est choisi dans le groupe constitué par Mn, Fe, Cu et Zn.

4. Procédé selon l'une quelconque des revendications précédentes, **caractérisé en ce que** le sel de métal de transition est un sel de Mn(II), Mn(IV), Fe(II), Fe(III), Cu(I), Cu (II) ou Zn(II), particulièrement de Fe(II) ou Fe(III).

5. Procédé selon l'une quelconque des revendications précédentes, **caractérisé en ce que** l'acide organique est choisi dans le groupe constitué par l'acide acétique, l'acide adipique, l'acide lactique, l'acide oxalique, l'acide citrique, particulièrement l'acide acétique.

6. Procédé selon l'une quelconque des revendications précédentes, **caractérisé en ce que** la quantité d'eau est supérieure à 90 % en poids, préférablement supérieure à 95 % du mélange réactionnel.

7. Procédé selon l'une quelconque des revendications précédentes, **caractérisé en ce que** la somme du poids
a) du composé de formule (II)
b) de l'oxygène
c) du sel de métal de transition, qui ne comprend aucun ligand polydentate lié à l'ion métallique
d) de l'acide organique
e) de l'eau
est supérieure à 95 %, préférablement supérieure à 98 %, plus préférablement supérieure à 99 %, du poids du mélange réactionnel complet au début de l'oxydation.

8. Procédé selon l'une quelconque des revendications précédentes, **caractérisé en ce que** le composé de formule (II) est présent en une quantité de 0,3 à 10 % en poids, particulièrement de 0,8 à 5,0 % en poids, préférablement de 0,8 à 2,5 % en poids, sur la base du poids du mélange réactionnel complet au début de l'oxydation.

9. Procédé selon l'une quelconque des revendications précédentes, **caractérisé en ce que** le métal de transition du sel de métal de transition est présent au début de l'oxydation en une quantité d'au moins 0,02 % en moles, particulièrement en une quantité de 0,02 à 2 % en moles, plus particulièrement en une quantité de 0,3 à 1,0 % en moles, sur la base de la quantité du composé de formule (II).

10. Procédé selon l'une quelconque des revendications précédentes, **caractérisé en ce que** l'oxydation a lieu à un pH de 5 à 6,8, particulièrement de 5,5 à 6, 2 .

11. Procédé selon l'une quelconque des revendications précédentes, **caractérisé en ce que** l'agent oxydant est O₂ sous la forme d'air, ou d'oxygène pur, préférablement sous la forme d'oxygène pur.

12. Procédé selon la revendication 10, **caractérisé en ce que** l'oxydation a lieu sous pression, particulièrement sous une pression de plus de 2 bars, préférablement de plus de 3 bars.

13. Procédé selon l'une quelconque des revendications précédentes, **caractérisé en ce que** le procédé est un procédé continu.
